# EUROPÄISCHE PATENTANMELDUNG

(11) **EP 3 957 261 A1**
(43) Veröffentlichungstag der Anmeldung: **23.02.2022**
(21) Anmeldenummer: 20191859.6
(22) Anmeldetag: 20.08.2020
(51) Int. Cl.: A61B 17/88

(54) **VORRICHTUNG ZUR BESTIMMUNG DER FLIESSFÄHIGKEIT EINES KNOCHENZEMENTTEIGS**

(71) Anmelder: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(57) **Zusammenfassung**

Die Erfindung betrifft eine Vorrichtung zur Bestimmung der Fließfähigkeit eines Knochenzementteigs, umfassend ein hohlzylinderförmiges erstes Leitungselement mit einem ersten Ende, durch das ein Knochenzementteig in das erste Leitungselement einbringbar ist, und einem zweiten Ende, wobei das zweite Ende zumindest eine axial verlaufende Durchführung aufweist, durch die der Knochenzementteig aus dem ersten Leitungselement förderbar ist.

Die Erfindung betrifft weiterhin ein Verfahren zur Bestimmung der Fließfähigkeit eines Knochenzementteigs mit einer Vorrichtung, umfassend ein hohlzylinderförmiges erstes Leitungselement mit einem ersten Ende, durch das ein Knochenzementteig in das erste Leitungselement einbringbar ist, und einem zweiten Ende, wobei das zweite Ende zumindest eine axial verlaufende Durchführung aufweist, durch die der Knochenzementteig aus dem ersten Leitungselement förderbar ist, wobei die Durchführung einen Innendurchmesser im Bereich von 0,1 bis 3 mm und ein Verhältnis einer Länge der Durchführung zum Innendurchmesser der Durchführung von 10:1 bis 20:1 aufweist.

## Beschreibung

Die Erfindung betrifft eine Vorrichtung zur Bestimmung der Fließfähigkeit eines Knochenzementteigs, umfassend ein hohlzylinderförmiges erstes Leitungselement mit einem ersten Ende, durch das ein Knochenzementteig in das erste Leitungselement einbringbar ist, und einem zweiten Ende, wobei das zweite Ende zumindest eine axial verlaufende Durchführung aufweist, durch die der Knochenzementteig aus dem ersten Leitungselement förderbar ist.

Die Erfindung betrifft weiterhin ein Verfahren zur Bestimmung der Fließfähigkeit eines Knochenzementteigs mit einer Vorrichtung, umfassend ein hohlzylinderförmiges erstes Leitungselement mit einem ersten Ende, durch das ein Knochenzementteig in das erste Leitungselement einbringbar ist, und einem zweiten Ende, wobei das zweite Ende zumindest eine axial verlaufende Durchführung aufweist, durch die der Knochenzementteig aus dem ersten Leitungselement förderbar ist, wobei die Durchführung einen Innendurchmesser im Bereich von 0,1 bis 3 mm und ein Verhältnis einer Länge der Durchführung zum Innendurchmesser der Durchführung von 10:1 bis 20:1 aufweist.

### Hintergrund der Erfindung

Die vollständige Arthroplastik ist ein weithin angewandtes Verfahren in der Orthopädie. Dabei wird infiziertes Gewebe, insbesondere Knochengewebe, entfernt und durch künstliche Prothesen aus Metall oder Kunststoff ersetzt. Üblicherweise werden die Prothesen mittels Knochenzement am verbleibenden, gesunden Knochengewebe fixiert. Insbesondere bei der Fixierung von Hüftprothesen im dafür vorbereiteten medullären Knochenkanal des Femurs besteht, durch die hohe mechanische Beanspruchung, das Risiko einer Lockerung der Prothese, üblicherweise im Bereich des Übergangs von Knochenzement zu Knochen. Um eine verbesserte Haftung zwischen Knochenzement und Knochen zu erreichen, wird der noch nicht ausgehärtete Knochenzement, der sogenannte Knochenzementteig, unter Krafteinwirkung in den Knochenkanal appliziert. Dies führt, bei ausreichender Fließfähigkeit des Knochenzementteigs, zu einem, zumindest teilweisen, Eindringen des Knochenzementteigs in die Spongiosa des Knochens und damit zu einer verbesserten Anbindung des ausgehärteten Knochenzements zum Knochen.

Üblicherweise werden zur Fixierung von Prothesen Polymethylmethacrylat-Knochenzemente verwendet. Dabei wird durch ein Vermischen einer Pulverkomponente mit einer Monomerkomponente zunächst ein Knochenzementteig angefertigt, welcher innerhalb einiger Minuten zu einem Knochenzement aushärtet. Der Operateur muss den Knochenzementteig in den Knochenkanal applizieren, bevor durch eine fortschreitende Polymerisation der Monomerkomponte die Fließfähigkeit des Knochenzementteigs derart weit herabgesetzt ist, dass ein Eindringen desselben in die Spongiosa unter Operationsbedingungen nicht mehr möglich ist. Dem Operateur steht somit nur ein begrenzter Verarbeitungszeitraum für den Knochenzementteig zur Verfügung. Erschwerend kommt hinzu, dass der Verarbeitungszeitraum, beispielsweise durch den Einfluss der Temperatur des Operationssaals, zeitlich variieren kann.

Es besteht daher im Markt ein Bedarf an Vorrichtungen zur Bestimmung der Fließfähigkeit von Knochenzementteigen.

In der EP 0 995 981 A wird eine Vorrichtung zur Bestimmung der Fließfähigkeit eines Knochenzementteigs beschrieben, wobei der Knochenzementteig aus einem Vorratsbehälter in eine Prüfleitung gefördert wird. Durch ein Beobachten, wie weit der Knochenzementteig aus dem Vorratsbehälter in die Prüfleitung gefördert wird, können Rückschlüsse über die Fließfähigkeit gezogen werden. Die beschriebene Prüfleitung ist nicht geeignet, um zu bestimmen, ob der Knochenzementteig in die Spongiosa eines Knochens applizierbar ist oder nicht. Dies stellt einen entscheidenden Nachteil der beschriebenen Prüfleitung dar, da nur bei einer geeigneten Fließfähigkeit eine feste Anbindung zwischen ausgehärtetem Knochenzement und Knochen, und damit eine feste Fixierung einer Prothese, möglich ist.

### Aufgaben

Eine Aufgabe der vorliegenden Erfindung ist es, einen oder mehrere der sich aus dem Stand der Technik ergebenden Nachteile zumindest teilweise zu überwinden.

Im Speziellen basiert die Erfindung auf dem Ziel, Vorrichtungen zum Bestimmen der Fließfähigkeit eines Knochenzementteigs bereitzustellen, mit denen bestimmbar ist, ob der Knochenzementteig eine Fließfähigkeit aufweist, um eine Applikation in die Spongiosa eines Knochens unter Operationsbedingungen zu ermöglichen. Die Vorrichtungen sollen ausgestaltet sein, die Fließfähigkeit von Knochenzementen unabhängig der Behältnisse, in denen der Knochenzementteig bereitgestellt wird, zu bestimmen.

Es ist eine weitere Aufgabe der Erfindung, ein Verfahren bereitzustellen, mit dem bestimmbar ist, ob ein Knochenzementteig in die Spongiosa eines Knochens applizierbar ist oder nicht.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.
|1| Vorrichtung zur Bestimmung der Fließfähigkeit eines Knochenzementteigs, umfassend ein hohlzylinderförmiges erstes Leitungselement mit einem ersten Ende, durch das ein Knochenzementteig in das erste Leitungselement einbringbar ist, und einem zweiten Ende,
   wobei das zweite Ende zumindest eine axial verlaufende Durchführung aufweist, durch die der Knochenzementteig aus dem ersten Leitungselement förderbar ist,
   **dadurch gekennzeichnet, dass**
   die Durchführung einen Innendurchmesser im Bereich von 0,1 mm bis 3 mm und ein Verhältnis einer Länge der Durchführung zum Innendurchmesser der Durchführung von 10:1 bis 20:1 aufweist, damit bei einem Fördern des Knochenzementteigs aus dem ersten Leitungselement durch die Durchführung mit einer Förderkraft von bis zu 2 kN (Kilonewton) bestimmbar ist, ob der Knochenzementteig eine Fließfähigkeit aufweist, um in die Spongiosa eines Knochens applizierbar zu sein.
|2| Vorrichtung nach Ausführungsform 1, **dadurch gekennzeichnet, dass** die Durchführung einen Innendurchmesser im Bereich von 0,2 mm bis 2 mm, bevorzugt im Bereich von 0,3 mm bis 1 mm, aufweist.
|3| Vorrichtung nach einer der vorhergehenden Ausführungsformen, **dadurch gekennzeichnet, dass** die Vorrichtung über das erste Ende reversibel, insbesondere durch Aufstecken, mit einem Vorratsbehälter für Knochenzementteige form- und/oder kraftschlüssig fluidleitend verbindbar ist.
|4| Vorrichtung nach Ausführungsform 3, **dadurch gekennzeichnet, dass** das erste Leitungselement vom ersten Ende in Richtung des zweiten Endes konisch verjüngt ausgestaltet ist, so dass die Vorrichtung reversibel durch Aufstecken mit einem Austragsschnorchel eines Vorratsbehälters für Knochenzementteige form- und/oder kraftschlüssig fluidleitend verbindbar ist.
|5| Vorrichtung nach einer der vorhergehenden Ausführungsformen, **dadurch gekennzeichnet, dass** die Vorrichtung einen separaten Kolben umfasst, der ausgestaltet ist reversibel axial in dem ersten Leitungselement verschiebbar zu sein, um einen Knochenzementteig aus dem ersten Leitungselement durch die zumindest eine Durchführung zu fördern.
|6| Vorrichtung nach einer der vorhergehenden Ausführungsformen, **dadurch gekennzeichnet, dass** die Vorrichtung ein zweites Leitungselement aufweist, welches mit dem ersten Leitungselement axial am zweiten Ende fluidleitend verbunden ist, um einen aus dem ersten Leitungselement durch die Durchführung geförderten Knochenzementteig aufzunehmen.
|7| Verfahren zur Bestimmung der Fließfähigkeit eines Knochenzementteigs mit einer Vorrichtung, umfassend
   ein hohlzylinderförmiges erstes Leitungselement mit einem ersten Ende, durch das ein Knochenzementteig in das erste Leitungselement einbringbar ist, und einem zweiten Ende, wobei das zweite Ende zumindest eine axial verlaufende Durchführung aufweist, durch die der Knochenzementteig aus dem ersten Leitungselement förderbar ist, wobei die Durchführung einen Innendurchmesser im Bereich von 0,1 bis 3 mm und ein Verhältnis einer Länge der Durchführung zum Innendurchmesser der Durchführung von 10:1 bis 20:1 aufweist,
   **zumindest aufweisend die Schritte**
   a) Bereitstellen eines Knochenzementteigs in einem Vorratsbehälter,
   b) Einbringen des Knochenzementteigs aus dem Vorratsbehälter durch das erste Ende in das erste Leitungselement,
   c) Ausüben einer Förderkraft von bis zu 2 kN, um den Knochenzementteig aus Richtung des ersten Endes durch die Durchführung aus dem ersten Leitungselement zu fördern, und
   d) Überprüfen, ob der Knochenzementteig durch die Durchführung gefördert wird.
|8| Verfahren nach Ausführungsform 7, **dadurch gekennzeichnet, dass**
   in Schritt b) das Einbringen des Knochenzementteigs in das erste Leitungselement durch ein reversibles fluidleitendes Verbinden der Vorrichtung mit einem Austragsschnorchel des Vorratsbehälters und anschließendem Betätigen eines mit dem Vorratsbehälter verbundenen Austragselements erfolgt, um den Knochenzementteig aus dem Vorratsbehälter über den Austragsschnorchel in das erste Leitungselement zu fördern, sowie
   in Schritt c) die Förderkraft über eine fortgeführte Betätigung des Austragselements ausgeübt wird.
|9| Verfahren nach Ausführungsform 7, **dadurch gekennzeichnet, dass**
   in Schritt b) das Einbringen des Knochenzementteigs in das erste Leitungselement durch eine Entnahme des Knochenzementteigs aus dem Vorratsbehälter und ein Einfüllen des entnommenen Knochenzementteigs durch das erste Ende in das erste Leitungselement, sowie
   in Schritt c) die Förderkraft mittels eines separaten Kolbens, der axial im ersten Leitungselement verschoben wird, ausgeübt wird.

### Allgemeines

In der vorliegenden Beschreibung beinhalten Bereichsangaben auch die als Grenzen genannten Werte. Eine Angabe der Art "m Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen parallele Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

### Ausführliche Beschreibung

Ein erster Gegenstand der Erfindung betrifft eine Vorrichtung zur Bestimmung der Fließfähigkeit eines Knochenzementteigs, umfassend ein hohlzylinderförmiges erstes Leitungselement mit einem ersten Ende, durch das ein Knochenzementteig in das erste Leitungselement einbringbar ist, und einem zweiten Ende, wobei das zweite Ende zumindest eine axial verlaufende Durchführung aufweist, durch die der Knochenzementteig aus dem ersten Leitungselement förderbar ist, dadurch gekennzeichnet, dass die Durchführung einen Innendurchmesser im Bereich von 0,1 mm bis 3 mm und ein Verhältnis einer Länge der Durchführung zum Innendurchmesser der Durchführung von 10:1 bis 20:1 aufweist, damit bei einem Fördern des Knochenzementteigs aus dem ersten Leitungselement durch die Durchführung mit einer Förderkraft von bis zu 2 kN bestimmbar ist, ob der Knochenzementteig eine Fließfähigkeit aufweist, um in die Spongiosa eines Knochens applizierbar zu sein.

Die Vorrichtung umfasst ein hohlzylinderförmiges erstes Leitungselement. Unter einem Leitungselement ist ein rohrartiges Gebilde zu verstehen, welches einen Innenraum und eine den Innenraum umgebende Leitungselementwand umfasst. Der Querschnitt des Leitungselements kann verschiedene Geometrien aufweisen. Beispielsweise kann der Querschnitt oval, quadratisch, fünfeckig, sechseckig, unregelmäßig oder kreisförmig ausgestaltet sein, wobei kreisförmig aufgrund der einfachen Fertigung und Handhabbarkeit bevorzugt ist.

Das erste Leitungselement weist ein erstes Ende auf, durch das ein Knochenzementteig in das erste Leitungselement, insbesondere in den Innenraum des ersten Leitungselements, förderbar ist. Das erste Ende kann dafür unterschiedlich ausgestaltet sein. Beispielsweise kann das erste Ende eine Verschlussklappe aufweisen, durch welche in einem geöffneten Zustand der Knochenzementteig in das erste Leitungselement befördert werden kann. Aufgrund der einfachen Bauweise ist es bevorzugt, dass das erste Ende eine kreisförmige Öffnung aufweist, welche im Wesentlichen einem Innendurchmesser des ersten Leitungselements entspricht, wobei die Öffnung durch die Leitungselementwand des Leitungselements gebildet wird.

Das erste Leitungselement weist ein zweites Ende mit zumindest einer axial verlaufenden Durchführung auf, durch welche ein Knochenzementteig aus dem Innenraum des ersten Leitungselement heraus förderbar ist. Die Durchführung dient dem Austragen eines Knochenzementteigs aus dem ersten Leitungselement. Unter einer Durchführung ist ein tunnelartiger Hohlraum zu verstehen, welcher das erste Leitungselement, insbesondere den Innenraum des ersten Leitungselement, mit einer Umgebung des ersten Leitungselements fluidleitend verbindet. Die Durchführung verläuft aus dem Innenraum des ersten Leitungselement durch das zweite Ende aus dem ersten Leitungselement heraus. Die Ausgestaltung des axialen Verlaufs der Durchführung kann dabei unterschiedlich ausgestaltet sein. Aufgrund der leichten Fertigungsweise ist es bevorzugt, dass die Durchführung parallel zur Längsachse der Vorrichtung verläuft. In einer Ausgestaltungsform weist die Vorrichtung genau eine Durchführung auf. Falls das zweite Ende genau eine Durchführung aufweist, ist es bevorzugt, dass die genau eine Durchführung zentral im zweiten Ende angeordnet ist. Der Innendurchmesser der Durchführung kann über die Länge der Durchführung unterschiedliche Werte annehmen, wobei es aufgrund einer besseren Bestimmung der Fließfähigkeit des Knochenzementteigs bevorzugt ist, dass der Innendurchmesser der Durchführung über zumindest 50 %, bevorzugt zumindest 70 %, weiter bevorzugt über zumindest 80 % der Gesamtlänge der Durchführung im Wesentlichen konstant ist. In einer Ausgestaltungsform ist der Innendurchmesser über die gesamte Länge der Durchführung im Wesentlichen konstant.

Das zweite Ende ist, bis auf die zumindest eine Durchführung, für den Knochenzementteig fluidleitend verschlossen, so dass ein Fördern aus dem ersten Leitungselement, insbesondere aus dem Innenraum des ersten Leitungselements, durch das zweite Ende nur über die zumindest eine Durchführung möglich ist.

Um mittels der Vorrichtung zu bestimmen, ob der verwendete Knochenzementteig eine geeignete Fließfähigkeit aufweist, um in die Spongiosa eines Knochens applizierbar zu sein, wird bei einem Fördern des Knochenzementteigs aus dem ersten Leitungselement, insbesondere aus dem Innenraum des ersten Leitungselements, durch die Durchführung eine maximale Förderkraft von 2 kN ausgeübt. Zur sicheren Verankerung zwischen Knochenzement und Knochen, sollte der Knochenzementteig zumindest teilweise, beispielsweise zumindest 2 mm, in die Spongiosa des Knochens appliziert werden. Die Eindringtiefe in die Spongiosa hängt dabei von der verwendeten Förderkraft ab, welche auf den Knochenzementteig während des Applizierens ausgeübt wird. Daher ist es vorteilhaft, wenn die Bestimmung der Fließfähigkeit des Knochenzementteigs mittels der Vorrichtung unter vergleichbaren Förderkraftbedingungen wie die Applikation in den Knochenkanal ausgeführt wird. Eine Förderkraft von 2 kN entspricht der maximalen Förderkraft handelsüblicher Austragselemente, wie beispielsweise einer handelsüblichen Austragspistole, welche dazu verwendet werden, den Knochenzementteig aus einem Vorratsbehälter, beispielsweise einer Kartusche, in einen dafür vorbereiteten Knochenkanal zu applizieren. Findet das Applizieren ohne Verwendung eines Austragselement statt, beispielsweise durch händisches Einbringen des Knochenzementteigs mittels Spatel durch den Operateur, kann üblicherweise keine höhere Förderkraft als 2 kN ausgeübt werden. Die Förderkraft zur Bestimmung der Fließfähigkeit sollte entsprechend den Verhältnissen des Applizierens angepasst werden. Eine maximale Förderkraft von 2 kN stellt somit die realistischen Bedingungen im Zuge einer Operation dar, welcher üblicherweise nicht überschritten werden.

Die Durchführung ist derart ausgestaltet, dass ein erfolgreiches Fördern des Knochenzementteigs durch die Durchführung mit einer Förderkraft von bis zu 2 kN dem Operateur anzeigt, dass der Knochenzementteig noch eine geeignete Fließfähigkeit aufweist, um in die Spongiosa des Knochens applizierbar zu sein. Findet hingegen bei Ausübung der maximalen Förderkraft von 2 kN kein Fördern oder ein Fördern nur bei einer höheren Förderkraft statt, zeigt dies dem Anwender, dass der Knochenzementteig keine geeignete Fließfähigkeit aufweist, um unter realistischen Operationsbedingungen in die Spongiosa des Knochens applizierbar zu sein.

Dafür weist die Durchführung zumindest bereichsweise, insbesondere über zumindest 50 %, bevorzugt über zumindest 70 %, weiter bevorzugt über zumindest 80 % der Gesamtlänge der Durchführung, einen Innendurchmesser im Bereich von 0,1 mm bis 3 mm, vorzugsweise im Bereich von 0,2 mm bis 2 mm, weiter bevorzugt im Bereich von 0,3 mm bis 1 mm, am bevorzugtesten im Bereich von 0,3 mm bis 0,5 mm auf. Der Innendurchmesser weist einen Maximalwert von 3 mm, bevorzugt von 2 mm, weiter bevorzugt von 1 mm und am bevorzugtesten von 0,5 mm auf. Der Innendurchmesser weist einen Minimalwert von 0,1 mm, bevorzugt 0,2 mm, weiter bevorzugt von 0,3 mm auf. Dies hat den Vorteil, dass nur Knochenzementteige durch eine derart ausgestaltete Durchführung mit einer Förderkraft von bis zu 2 kN förderbar sind, die bei Ausübung einer vergleichbaren Kraft ebenso zumindest teilweise in die Spongiosa eines Knochens einbringbar sind. Ist der Knochenzementteig bei einem Innendurchmesser von bis zu 3 mm nicht durch die Durchführung förderbar, ist die Fließfähigkeit zu gering und eine Applikation in die Spongiosa des Knochens zur festen Fixierung einer Prothese nicht möglich. Die Durchführung weist zumindest einen Innendurchmesser von 0,1 mm auf, damit die Bestimmung der Fließfähigkeit derart schnell erfolgen kann, dass die mögliche Verarbeitungszeit des Knochenzements durch den Operateur nicht durch die Bestimmung unnötig verkürzt wird und möglichst viel Zeit für die Applikation in einen Knochenkanal zur Verfügung steht. Der Innendurchmesser der Durchführung kann über die gesamte Länge der Durchführung konstant sein oder variieren, wobei es aufgrund einer besseren Bestimmung der Fließfähigkeit des Knochenzementteigs bevorzugt ist, dass der Innendurchmesser der Durchführung über zumindest 50 %, bevorzugt zumindest 70 %, weiter bevorzugt über zumindest 80 % der Gesamtlänge der Durchführung im Wesentlichen konstant ist. In einer Ausgestaltungsform weist die Durchführung an der dem ersten Ende zugewandten Seite einen trompetenförmigen Durchführungseingang auf, welcher einen größeren Innendurchmesser als 3 mm aufweisen kann, wobei zumindest 50 % der Gesamtlänge der Durchführung einen Innendurchmesser im Bereich von 0,1 bis 3 mm besitzt. Ein trompetenförmiger Durchführungseingang hat den Vorteil, dass ein Knochenzementteig leichter zur Durchführung hingeleitet wird und das Risiko einer Verstopfung der Durchführung verringert wird.

Die Durchführung weist darüber hinaus ein Verhältnis einer Länge der Durchführung zum Innendurchmesser der Durchführung von 10:1 bis 20:1, bevorzugt von 12:1 bis 18:1, weiter bevorzugt von 14:1 bis 16:1, am bevorzugtesten von 15:1 auf. Die Länge der Durchführung entspricht somit zumindest 10-mal, bevorzugt zumindest 12-mal, weiter bevorzugt zumindest 14-mal dem Innendurchmesser der Durchführung. Die Länge der Durchführung entspricht höchstens 20-mal, bevorzugt höchstens 18-mal, weiter bevorzugt höchstens 16-mal dem Innendurchmesser der Durchführung. Am bevorzugtesten entspricht die Länge der Durchführung 15-mal dem Innendurchmesser der Durchführung. Ist ein Knochenzementteig durch eine derart ausgestaltete Durchführung mit einer Förderkraft von bis zu 2 kN förderbar, ist sichergestellt, dass der Knochenzementteig zum Zeitpunkt der Bestimmung in die Spongiosa eines Knochens applizierbar ist und darüber hinaus eine ausreichend zeitliche Reserve für den Operateur zur Verfügung steht, um den Knochenzementteig in einen Knochenkanal zu applizieren und der Knochenzementteig dabei immer noch eine ausreichend hohe Fließfähigkeit aufweist, um in die Spongiosa eines Knochens zumindest teilweise einzudringen. Die zeitliche Reserve für den Operateur beträgt bei handelsüblichen Knochenzementen für das Verhältnis der Länge der Durchführung zum Innendurchmesser der Durchführung von 10:1 beispielsweise mindestens 1 Minute und verlängert sich mit größerem Verhältnis, beispielsweise auf bis zu 5 Minuten bei einem Verhältnis von 20:1.

Des Leitungselement und/oder die gesamte Vorrichtung kann unterschiedliche Materialien oder Materialkombinationen aufweisen. Beispielsweise kann die Vorrichtung Kunststoff aufweisen oder aus Kunststoff bestehen. Bevorzugt handelt es sich bei dem Kunststoff um einen transparenten Kunststoff, da der Anwender auf diese Weise die Fließfähigkeit des Knochenzementteigs einfach und direkt optisch bestimmen kann.

Der Innendurchmesser des ersten Leitungselements kann unterschiedliche Werte annehmen.

Eine Ausgestaltungsform der Vorrichtung ist dadurch gekennzeichnet, dass die Vorrichtung zumindest im Bereich des ersten Endes einen derartigen Innendurchmesser aufweist, dass die Vorrichtung reversibel form- und/oder kraftschlüssig, insbesondere durch Aufstecken, mit einem Vorratsbehälter für Knochenzementteige, insbesondere an einer Austragsöffnung des Vorratsbehälters für Knochenzementteige, fluidleitend verbindbar ist. Dies erlaubt ein direktes Fördern eines Knochenzementteiges aus einem Vorratsbehälter in die Vorrichtung mit anschließender Bestimmung der Fließfähigkeit des Knochenzementteigs. Somit kann der Knochenzementteig zur Applikation bereitgestellt werden und der Operateur kann vor dem Applizieren in einen Knochenkanal schnell und einfach durch fluidleitendes Verbinden der Vorrichtung mit dem Vorratsbehälter bestimmen, ob der Knochenzementteig noch eine geeignete Fließfähigkeit zur Applikation in die Spongiosa des Knochens aufweist oder nicht. Nach erfolgter Bestimmung kann die Vorrichtung ebenso schnell und einfach wieder von dem Vorratsbehälter gelöst und der Knochenzement appliziert werden.

Um mit einem Vorratsbehälter für Knochenzementteige fluidleitend verbindbar zu sein, kann das erste Leitungselement, insbesondere das erste Ende, auf unterschiedliche Weise ausgestaltet sein.

Eine Ausgestaltungsform der Vorrichtung ist dadurch gekennzeichnet, dass das erste Leitungselement vom ersten Ende in Richtung des zweiten Endes konisch verjüngt ausgestaltet ist, so dass die Vorrichtung über das erste Ende reversibel durch Aufstecken mit einem Austragsschnorchel eines Vorratsbehälters für Knochenzementteige form- und/oder kraftschlüssig fluidleitend verbindbar ist. Ein Austragsschnorchel ist ein mit einen Vorratsbehälter für Knochenzementteige fluidleitend verbindbares, rohrartiges Mittel, welches eine zielgenaue Applikation eines Knochenzementteigs aus dem Vorratsbehälter an die gewünschte Stelle, insbesondere in einen Knochenkanal, erlaubt. Durch die konische Verjüngung weist die Vorrichtung, insbesondere das erste Leitungselement, am ersten Ende einen größeren Innendurchmesser als am zweiten Ende auf. Das konisch verjüngte Leitungselement kann schnell und einfach reversibel auf einen Austragsschnorchel aufgesteckt werden, wobei die konische Form eine einfache und sichere Befestigung der Vorrichtung an einem Austragsende für Knochenzementteige des Austragsschnorchels erlaubt. Dies erlaubt ein direktes Fördern eines Knochenzementteiges aus einem Vorratsbehälter über einen Austragsschnorchel in die Vorrichtung mit anschließender Bestimmung der Fließfähigkeit des Knochenzementteigs. Somit kann der Knochenzementteig zur Applikation bereitgestellt werden und der Operateur kann vor dem Applizieren in einen Knochenkanal schnell und einfach durch Aufstecken der Vorrichtung bestimmen, ob der Knochenzementteig noch eine geeignete Fließfähigkeit zur Applikation in die Spongiosa des Knochens aufweist oder nicht. Nach erfolgter Bestimmung kann die Vorrichtung ebenso schnell und einfach wieder von dem Austragsschnorchel gelöst und der Knochenzement appliziert werden.

Die Förderkraft zum Fördern eines Knochenzementteigs durch die Durchführung kann auf unterschiedliche Weise ausgeübt werden. In einer Ausgestaltungsform wird die Förderkraft mittels eines Austragselements, insbesondere einer Austragspistole, ausgeübt.

Eine Ausgestaltungsform der Vorrichtung ist dadurch gekennzeichnet, dass die Vorrichtung einen separaten Kolben umfasst, der ausgestaltet ist, reversibel axial innerhalb des ersten Leitungselement verschiebbar zu sein, um einen Knochenzementteig aus dem ersten Leitungselement durch die zumindest eine Durchführung zu fördern. In einer Ausgestaltungsform weist der Kolben dafür einen Außendurchmesser auf, welcher im Wesentlichen dem Innendurchmesser des ersten Leitungselements entspricht, beziehungsweise, bei konischer Ausgestaltungsform des ersten Leitungselement, im Wesentlichen dem geringsten Innendurchmesser des ersten Leitungselements entspricht. Der Operateur kann den Kolben verwenden, um, nach Einbringen einer Probe eines Knochenzementteigs in das erste Leitungselement, durch axiales Verschieben des Kolbens vom ersten Ende in Richtung des zweiten Endes eine Förderkraft auf den Knochenzementteig auszuüben, um die Fließfähigkeit des Knochenzementteigs zu bestimmen. Ein Vorteil ist, dass eine Bestimmung der Fließfähigkeit eines Knochenzementteigs nicht von einer Verwendung eines speziellen Vorratsbehälters für Knochenzementteige oder eines speziellen Austragselements, beispielsweiser einer Austragspistole, abhängig ist, sondern der Knochenzementteig in beliebigen Behältnissen, beispielsweise in einer Schüssel oder einem Topf, bereitgestellt werden kann. Somit erhöhen sich die Verwendungsmöglichkeiten der Vorrichtung.

Eine Ausgestaltungform der Vorrichtung ist dadurch gekennzeichnet, dass die Vorrichtung ein zweites Leitungselement aufweist, welches axial am zweiten Ende mit dem ersten Leitungselement verbunden, insbesondere fluidleitend verbunden, ist, um einen aus dem ersten Leitungselement durch die Durchführung geförderten Knochenzementteig aufzunehmen. Die Verbindung zwischen ersten Leitungselement und zweitem Leitungselement kann auf unterschiedliche Weise realisiert werden. In einer Ausgestaltungsform sind erstes Leitungselement und zweites Leitungselement beispielsweise über eine Schraubverbindung, über eine Steckverbindung oder über eine Klebeverbindung miteinander verbunden. In einer weiteren Ausgestaltungsform sind erstes Leitungselement und zweites Leitungselement einteilig ausgeformt. Durch die Aufnahme des Knochenzementteig durch das zweite Leitungselement ist sichergestellt, dass der aus dem ersten Leitungselement ausgetragenen Knochenzementteig nicht unkontrolliert in die Umgebung der Vorrichtung, insbesondere nicht unkontrolliert einen Operationssaal, mit Knochenzement kontaminiert. Dies erhöht zum einen den Anwendungskomfort durch den Operateur, als auch die Anwendungssicherheit der Vorrichtung.

Ein zweiter Gegenstand der vorliegenden Erfindung betrifft ein Verfahren zur Bestimmung der Fließfähigkeit eines Knochenzementteigs mit einer Vorrichtung, umfassend ein hohlzylinderförmiges erstes Leitungselement mit einem ersten Ende, durch das ein Knochenzementteig in das erste Leitungselement einbringbar ist, und einem zweiten Ende, wobei das zweite Ende zumindest eine axial verlaufende Durchführung aufweist, durch die der Knochenzementteig aus dem ersten Leitungselement förderbar ist, wobei die Durchführung einen Innendurchmesser im Bereich von 0,1 bis 3 mm und ein Verhältnis einer Länge der Durchführung zum Innendurchmesser der Durchführung von 10:1 bis 20:1 aufweist, zumindest aufweisend die Schritte
a) Bereitstellen eines Knochenzementteigs in einem Vorratsbehälter,
b) Einbringen des Knochenzementteigs aus dem Vorratsbehälter durch das erste Ende in das erste Leitungselement,
c) Ausüben einer Förderkraft von bis zu 2 kN, um den Knochenzementteig aus Richtung des ersten Endes durch die Durchführung aus dem ersten Leitungselement zu fördern, und
d) Überprüfen, ob der Knochenzementteig durch die Durchführung gefördert wird.

Eine weitere Ausgestaltungsform des Verfahrens ist dadurch gekennzeichnet, dass in Schritt b) des Verfahrens das Einbringen des Knochenzements in das erste Leitungselement durch ein reversibles fluidleitendes Verbinden der Vorrichtung, insbesondere des ersten Endes des ersten Leitungselements, mit einem Austragsschnorchel des Vorratsbehälters und anschließendem Betätigen eines mit dem Vorratsbehälter verbundenen Austragselements erfolgt. Das fluidleitende Verbinden der Vorrichtung kann auf unterschiedliche Weise stattfinden. In einer Ausgestaltungsform wird die Vorrichtung beispielsweise über einen Schlauch mit dem Austragsschnorchel verbunden, über den der Knochenzement in das erste Leitungselement gefördert werden kann. Aufgrund des geringen Arbeitsaufwandes und der geringen Anfälligkeit für Anwendungsfehler, ist es bevorzugt, wenn die Vorrichtung mit dem ersten Ende auf den Austragsschnorchel aufgesteckt wird. Dies sorgt für eine direkt und einfache fluidleitende Verbindung, welche nach der Bestimmung der Fließfähigkeit des Knochenzementteigs ohne größere Umbauten durch Abziehen wieder gelöst werden kann, so dass der Knochenzementteig in einen Knochenkanal appliziert werden kann. Das Austragselement, insbesondere eine Austragspistole, verbringt bei einer Betätigung den Knochenzementteig aus dem Vorratsbehälter über den Austragsschnorchel in das erste Leitungselement, insbesondere in den Innenraum des ersten Leitungselement. Bei der Verwendung einer Austragspistole geschieht dies beispielsweise über die Betätigung des Abzugs der Austragspistole. Weiterhin wird in dieser Ausgestaltungsform des Verfahrens in Schritt c) das Ausüben der Förderkraft, ebenso wie das Fördern des Knochenzementteigs in die Vorrichtung, über eine Betätigung des Austragselements, insbesondere einer Austragspistole, ausgeführt. Ein Vorteil ist dabei, dass die Förderkraft durch das Austragselement definiert vorgegeben ist und menschliche Fehler bei der Durchführung des Verfahrens möglichst ausgeschlossen sind.

Eine Ausgestaltungform des Verfahrens ist dadurch gekennzeichnet, dass in Schritt b) das Einbringen des Knochenzementteigs in das erste Leitungselement durch eine Entnahme des Knochenzementteigs aus dem Vorratsbehälter und ein Einfüllen des entnommenen Knochenzementteigs durch das erste Ende in das erste Leitungselement ausführt wird. Die Entnahme und das Einfüllen des Knochenzements können auf unterschiedliche Weise realisiert werden. In einer Ausgestaltungsform wird der Knochenzement händisch vom Operateur aus dem Vorratsbehälter, beispielsweise mittels eines Spatels oder Löffels, entnommen und in die Vorrichtung verbracht. Weiterhin wird in dieser Ausgestaltungsform des Verfahrens in Schritt c) die Förderkraft mittels eines separaten Kolbens, der axial im ersten Leitungselement vom ersten Ende in Richtung des zweiten Endes verschoben wird, ausgeübt. Der Kolben ist ausgestaltet, vom Operateur per Hand gehalten und axial innerhalb des ersten Leitungselements verschoben zu werden, um den Knochenzementteig aus dem ersten Leitungselement durch die Durchführung zu fördern.
Ein Vorteil ist, dass auf diese Weise das Verfahren zur Bestimmung der Fließfähigkeit mit unterschiedlichsten Vorratsbehältern durchgeführt werden kann. Das derartige Verfahren hängt nicht von der Verwendung eines bestimmten Vorratsbehälters oder eines mit dem Vorratsbehälter verbundenen Austragselements ab, sondern kann beispielsweise auch mit Knochenzementteigen durchgeführt werden, welche in einem Topf oder einer Schüssel bereitgestellt und mittels Spatel in einen Knochen appliziert werden.

Die Vorrichtung dient der Bestimmung der Fließfähigkeit eines Knochenzementteigs. Ein Knochenzementteig stellt die noch nicht ausgehärtete pastöse Form eines Knochenzements dar. Unter einem Knochenzement wird eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Bevorzugt handelt es sich bei Knochenzementen um Polymethylmethacrylat-Knochenzemente (PMMA Knochenzemente). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzement, der auch als Knochenzementteig bezeichnet wird. Dabei wird eine Polymerisation des Monomers durch Radikale eingeleitet. Mit fortschreitender Polymerisation des Monomers erhöht sich die Viskosität des Knochenzements, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulvers zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Die Partikelgröße des partikulären Polymethylmethacrylat und/oder des partikulären Polymethylmethacrylat-Copolymers des Knochenzementpulvers kann beispielsweise der Siebfraktion kleiner 150 µm, bevorzugt kleiner 100 µm, entsprechen.

Das hydrophile Additiv kann partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Der Initiator kann Dibenzoylperoxid enthalten oder aus Dibenzoylperoxid bestehen.

Unter einem Röntgenopaker versteht man eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen.

Die pharmazeutisch aktive Substanz kann ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind Gentamicin, Clindamycin und Vancomycin.

Die Monomerflüssigkeit kann das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

### Beispiele

Die Erfindung wird im Folgenden durch Beispiele weiter beispielhaft illustriert. Die Erfindung ist nicht auf die Beispiele beschränkt.

### Figuren

Es zeigen
- Fig. 1: einen schematischen Querschnitt einer Vorrichtung zur Bestimmung der Fließfähigkeit eines Knochenzementteigs,
- Fig. 2: einen schematischen Querschnitt der Vorrichtung aus Figur 1 gefüllt mit einem Knochenzementteig,
- Fig. 3: einen schematischen Teilquerschnitt der Vorrichtung aus Figur 1, angebracht an einem Vorratsbehälter für Knochenzementteige, und
- Fig. 4: ein Verfahren zur Bestimmung der Fließfähigkeit eines Knochenzementteigs mittels einer Vorrichtung.

### Beschreibung der Figuren

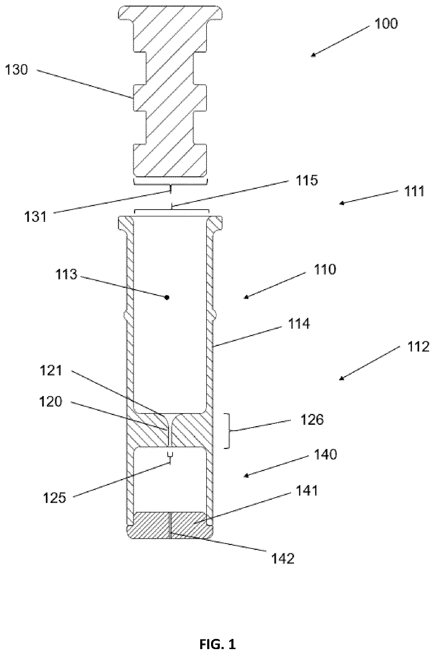

Figur 1 zeigt eine Vorrichtung 100 zur Bestimmung der Fließfähigkeit eines Knochenzementteigs, wobei die Vorrichtung aus mehreren Bauteilen aufgebaut ist. Die Vorrichtung 100 weist ein hohlzylinderförmiges erstes Leitungselement 110 mit einem Innenraum 113 und einer den Innenraum umgebenden rohrartigen Wandung 114 auf. Das erste Leitungselement 110 weist ein erstes Ende 110 und ein dem ersten Ende axial entgegengesetztes zweites Ende 112 auf.

Das erste Ende 111 ist offen ausgestaltet, so dass ein Knochenzementteig in das erste Leitungselement 110 einfüllbar ist.

Am zweiten Ende 112 weist das erste Leitungselement 110 eine Durchführung 120 auf. Die Durchführung erstreckt sich axial aus dem ersten Leitungselement 110, insbesondere aus dem Innenraum 113 des ersten Leitungselements 110, heraus. In der gezeigten Ausgestaltungsform der Vorrichtung 100 erstreckt sich die Durchführung aus dem ersten Leitungselement 110, insbesondere aus dem Innenraum des ersten Leitungselements 110, in ein hohlzylinderförmiges zweites Leitungselement 140, so dass erstes Leitungselement 110 und zweites Leitungselement 140 fluidleitend miteinander verbunden sind. Das zweite Leitungselement 140 dient dazu, einen aus dem ersten Leitungselement 110 durch die Durchführung 120 geförderten Knochenzementteig aufzunehmen, so dass der Knochenzementteig nicht unkontrolliert in die Umgebung der Vorrichtung 100 abgegeben wird und so zu Kontaminationen, insbesondere eines Operationssaals, führen kann. In einer weiteren, nicht gezeigten, Ausgestaltungsform der Vorrichtung 100 mündet die Durchführung 120 nicht in einem zweiten Leitungselement, sondern in der Umgebung der Vorrichtung 100.

In der gezeigten Ausführungsform ist die Durchführung 120 an einem dem Innenraum 113 des ersten Leitungselements 110 zugewandten Durchführungseingang 121 trompetenförmig ausgestaltet, wobei über sich der Innendurchmesser 125 der Durchführung 120 in Richtung des zweiten Endes 112 verjüngt. Ein Vorteil eines trompetenförmigen Durchführungseingangs 121 ist, dass ein Zuleiten eines Knochenzementteigs aus dem ersten Leitungselement 110 in die Durchführung 120 erleichtert ist und das Risiko einer Verstopfung der Durchführung 120 verringert ist. In weiteren, nicht gezeigten, Ausgestaltungsformen weist die Durchführung 120 einen trichterförmigen Durchgangseingang 121 oder einen über eine gesamte Länge 126 der Durchführung 120 konstanten Innendurchmesser 125 auf.

Das erste Leitungselement 110 ist axial mit dem zweiten Leitungselement 140 fluidleitend über die Durchführung 120 verbunden. In der gezeigten Ausgestaltungsform sind erste Leitungselement 110 und zweites Leitungselement einteilig ausgestaltet. In weiteren, nicht gezeigten, Ausgestaltungsformen sind das erste Leitungselement 110 und das zweite Leitungselement 140 form- und/oder kraftschlüssig miteinander axial verbunden. Das zweite Leitungselement 140 weist an einem dem ersten Leitungselement 110 abgewandten axialen Ende ein Verschlusselement 141 mit einer Verschlusselementöffnung 142 auf. Die Verschlusselementöffnung 142 ist ausgestaltet, um einen Gasdruck, welcher sich beim Fördern eines Knochenzementteigs aus dem ersten Leitungselement 110 in das zweite Leitungselement 140 bildet, aus dem zweiten Leitungselement 140 abzulassen und gleichzeitig den Knochenzement innerhalb des zweiten Leitungselement 140 zu verwahren. Dazu weist die Verschlusselementöffnung 142 in der gezeigten Ausgestaltungsform der Vorrichtung 100 einen geringeren Innendurchmesser als die Durchführung 120 auf.

Die Vorrichtung 100 weist einen separaten Kolben 130 auf. Der Kolben 130 weist einen Kolbenaußendurchmesser 131 auf, welcher im Wesentlichen einem Innendurchmesser 115 des ersten Leitungselements 110 entspricht, so dass der Kolben 130 reversibel axial innerhalb des ersten Leitungselements 110 verschiebbar ist. Ein axiales Verschieben des Kolbens 130 innerhalb des ersten Leitungselements 110 in Richtung des zweiten Endes 112 führt, bei mit einem Knochenzementteig befüllten Innenraum 114, zum Ausüben einer Förderkraft, wodurch der Knochenzementteig, bei geeigneter Fließfähigkeit, durch die Durchführung 130 gefördert wird. Das Verschieben des Kolbens 130 wird vorzugsweise händisch durch einen Operateur durchgeführt, um die Fließfähigkeit eines Knochenzementteigs zu bestimmen.

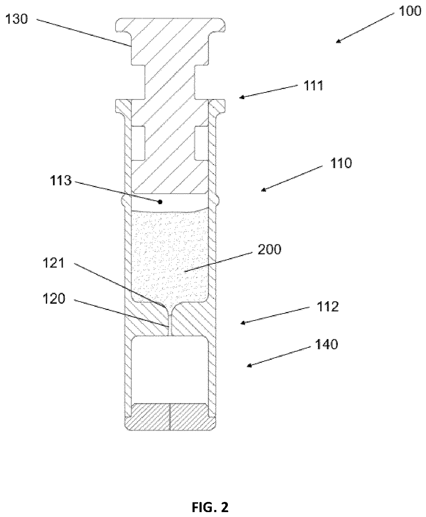

**Figur 2** zeigt die Vorrichtung 100 aus Figur 1 mit einem in das erste Leitungselement 110, insbesondere in den Innenraum 113 des ersten Leitungselements 100, eingebrachten Knochenzementteig 200. Der Kolben 130 ist zum Teil durch das erste Ende 111 in das erste Leitungselement 110 axial eingeschoben und der Knochenzementteig bereits teilweise in den trompetenförmigen Durchführungseingang 121 eingebracht. Durch ein fortgeführtes Verschieben des Kolbens 130 in Richtung des zweiten Endes 112 wird eine Förderkraft auf den Knochenzementteig 200 ausgeübt, so dass bestimmbar ist, ob der Knochenzementteig 200 eine geeignete Fließfähigkeit besitzt, um in die Spongiosa eines Knochens applizierbar zu sein. Falls der Knochenzementteig 200 durch die Durchführung 120 förderbar ist, und somit eine geeignete Fließfähigkeit zur Applikation in die Spongiosa eines Knochens vorliegt, dient das zweite Leitungselement 140 zur Lagerung des Knochenzementteigs 200, um eine Kontamination der Umgebung der Vorrichtung 100, insbesondere des Operationssaals oder des Operateurs, zu verhindern. Falls ein Fördern des Knochenzementteigs 200 durch die Durchführung 120 nicht möglich ist, und somit der Knochenzementteig 200 keine geeignete Fließfähigkeit aufweist, um ein die Spongiosa eines Knochens applizierbar zu sein, verbleibt der Knochenzementteig 200 innerhalb des ersten Leitungselements 110.

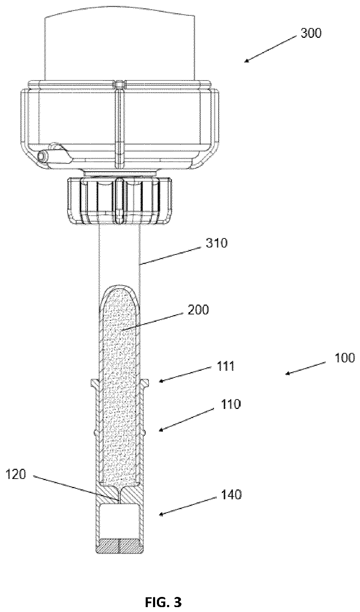

**Figur 3** zeigt die Vorrichtung 100 aus Figur 1 und 2 fluidleitend verbunden mit einem Vorratsbehälter 300 für einen Knochenzementteig 200. Die Vorrichtung 100 ist mit dem ersten Ende 111 auf einen Austragsschnorchel 310 des Vorratsbehälter 300 aufgesteckt und damit fluidleitend verbunden. Der Knochenzementteig 200 ist teilweise aus dem Vorratsbehälter 300 über den Austragsschnorchel 310 in das erste Leitungselement 110 eingebracht. Durch ein fortgeführtes Fördern des Knochenzementteigs 200 aus dem Vorratsbehälter 300 in das erste Leitungselement 110, beispielsweise mittels eines mit dem Vorratsbehälter 300 verbundenes Austragselements (nicht gezeigt), wird eine Förderkraft auf den Knochenzementteig 200 ausgeübt, so dass bestimmbar ist, ob der Knochenzementteig 200 eine geeignete Fließfähigkeit besitzt, um in die Spongiosa eines Knochens applizierbar zu sein. Falls der Knochenzementteig 200 durch die Durchführung 120 förderbar ist, und somit eine geeignete Fließfähigkeit zur Applikation in die Spongiosa eines Knochens vorliegt, dient das zweite Leitungselement 140 zur Lagerung des Knochenzementteigs 200, um eine Kontamination der Umgebung der Vorrichtung 100, insbesondere des Operationssaals oder des Operateurs, zu verhindern. Falls ein Fördern des Knochenzementteigs 200 durch die Durchführung 120 nicht möglich ist, und somit der Knochenzementteig 200 keine geeignete Fließfähigkeit aufweist, um ein die Spongiosa eines Knochens applizierbar zu sein, verbleibt der Knochenzementteig 200 innerhalb des ersten Leitungselements 110.

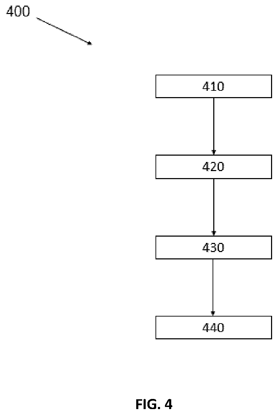

**Figur 4** zeigt ein Flussdiagrimm enthaltend die Schritte 410 bis 440 eines Verfahrens 400 zur Bestimmung der Fließfähigkeit eines Knochenzementteigs 200 mittels der Vorrichtung 100 umfassend das hohlzylinderförmiges erstes Leitungselement 110 mit dem ersten Ende 111, durch das der Knochenzementteig 200 in das erste Leitungselement 110 einbringbar ist, und dem zweiten Ende 112, wobei das zweite Ende 112 zumindest die axial verlaufende Durchführung 120 aufweist, durch die der Knochenzementteig 200 aus dem ersten Leitungselement 110 förderbar ist, wobei die Durchführung 120 einen Innendurchmesser 125 im Bereich von 0,1 bis 3 mm und ein Verhältnis einer Länge 125 der Durchführung 120 zum Innendurchmesser 125 der Durchführung 120 von 10:1 bis 20:1 aufweist.

In einem Schritt 410 wird der Knochenzementteig 200 in einem Vorratsbehälter 300 bereitgestellt. In einer Ausgestaltungsform des Verfahrens 400 wird der Knochenzementteig 200 in einem mit einem Austragsschnorchel 310 ausgestatteten Vorratsbehälter 300 bereitgestellt. In einer weiteren Ausgestaltungsform des Verfahrens 400 wird der Knochenzementteig 200 in einem Vorratsbehälter 300 ohne Austragsschnorchel 310, beispielsweise in einer Schüssel oder einem Topf, bereitgestellt.

In einem Schritt 420 wird der Knochenzementteig 200 aus dem Vorratsbehälter 300 durch das erste Ende 111 in das erste Leitungselement 110 eingebracht. In einer Ausgestaltungsform des Verfahrens 400 wird der Knochenzementteig 200 über einen mit dem Vorratsbehälter 300 fluidleitend verbundenen Austragsschnorchel 310 in das erste Leitungselement 110 eingebracht. Bevorzugt wird dazu die Vorrichtung 100, insbesondere mit dem ersten Ende 110, auf den Austragsschnorchel 310 aufgestülpt, so dass das erste Leitungselement 110 reversibel fluidleitend mit dem Austragsschnorchel 310 verbunden ist. Um den Knochenzementteig 200 aus dem Vorratsbehälter 300 in das erste Leitungselement zu verbringen, wird eine mit dem Vorratsbehälter 300 verbundenes Austragselement, beispielsweise eine Austragspistole, betätigt. Durch die reversible fluidleitende Verbindung zwischen der Vorrichtung 100 und dem Vorratsbehälter 300 ist das Einbringen des Knochenzements 200 schnell, einfach und ohne zusätzliche Werkzeuge möglich. In einer weiteren Ausgestaltungsform des Verfahrens 400 wird der Knochenzementteig 200 durch eine Entnahme, beispielsweise mittels Spatel oder Löffel, aus dem Vorratsbehälter 300 und Überführung sowie Einfüllen in das erste Leitungselement 110 durchgeführt, also ohne direkte fluidleitende Verbindung zwischen Vorrichtung 100 und Vorratsbehälter 300. Dadurch ist es möglich, jede Art von Vorratsbehälter 300 zum Bereitstellen des Knochenzementteigs 200 zu verwenden und ist nicht auf eine bestimmte Ausgestaltungsform festgelegt.

In einem Schritt 430 wird eine Förderkraft von bis zu 2 kN auf den Knochenzementteig 200 in Richtung des ersten Endes 111 ausgeübt, um den Knochenzementteig 200 aus dem ersten Leitungselement 110 durch die Durchführung 200 zu fördern. Ist die Vorrichtung 100 reversibel fluidleitend mit einem Austragsschnorchel 310 des Vorratsbehälters 300 verbunden ist, wird in einer Ausgestaltungsform des Verfahrens 400 die Förderkraft durch eine fortgeführte Betätigung des Austragselements auf den Knochenzementteig 200 ausgeübt. Auf diese Weise sind keine weiteren Umbauten oder zusätzliche Gerätschaften notwendig, was die Anwendung des Verfahrens 400 für den Operateur vereinfacht. Ist die Vorrichtung 100 nicht fluidleitend mit dem Vorratsbehälter 300 verbunden, wird in einer Ausgestaltungsform des Verfahrens 400 die Förderkraft mittels des separaten Kolbens 130 der Vorrichtung 100, der axial im ersten Leitungselement in Richtung der Durchführung 120 durch den Operateur verschoben wird, ausgeübt. Durch die Verwendung des separaten Kolbens 130 kann die Bestimmung der Fließfähigkeit des Knochenzementteigs 200 unabhängig vom strukturellen Aufbau des Vorratsbehälters 300 ausgeführt werden, wodurch das Verfahren 400 flexibel einsetzbar ist.

In einem Schritt 440 wird überprüft, ob der Knochenzementteig 200 durch die Durchführung 120 mittels der ausgeübten Förderkraft gefördert wird. Findet ein Fördern statt, weist der Knochenzementteig 200 eine derartige Fließfähigkeit auf, um in die Spongiosa eines Knochens zumindest teilweise applizierbar zu sein. Findet kein Fördern des Knochenzementteigs 200 durch die Durchführung 120 statt, ist der Knochenzementteig 200 nicht geeignet in die Spongiosa eines Knochens applizierbar zu sein und sollte vom Operateur nicht mehr in den Knochenkanal eingebracht werden. In einer bevorzugten Ausgestaltungsform des Verfahrens 400 findet das Überprüfen optisch durch den Operateur statt. Vorzugsweise umfasst die Vorrichtung 100 dazu zumindest im Bereich der Durchführung 120 einen optisch transparenten Kunststoff, so dass der Operateur direkt und ohne weitere Schritte, wie beispielsweise ein Öffnen der Vorrichtung, erkennen kann, ob der Knochenzementteig 200 durch die Durchführung 120 gefördert wird.

Die in den Ansprüchen, der Beschreibung und in den Figuren offenbarten Merkmale können für verschiedene Ausgestaltungsformen der beanspruchten Erfindung sowohl getrennt als auch in beliebiger Kombination miteinander wesentlich sein. Die für die Vorrichtung offenbarten Merkmale sind auch für das Verfahren offenbart und umgekehrt.

### Bezugszeichen

- 100: Vorrichtung
- 110: erstes Leitungselement
- 111: erstes Ende
- 112: zweites Ende
- 113: Innenraum des erste Leitungselements
- 114: Wandung des ersten Leitungselements
- 115: Innendurchmesser des ersten Leitungselements
- 120: Durchführung
- 121: Durchführungseingang
- 125: Innendurchmesser der Durchführung
- 126: Länge der Durchführung
- 130: Kolben
- 131: Kolbenaußendurchmesser
- 140: zweites Leitungselement
- 141: Verschlusselement
- 142: Verschlusselementöffnung
- 200: Knochenzementteig
- 300: Vorratsbehälter für Knochenzementteige
- 310: Austragsschnorchel
- 400: Verfahren zur Bestimmung der Fließfähigkeit eines Knochenzementteigs
- 410: Bereitstellen eines Knochenzementteigs
- 420: Einbringen des Knochenzementteigs in das erste Leitungselement
- 430: Ausüben einer Förderkraft
- 440: Überprüfen

## Patentansprüche

1. Vorrichtung (100) zur Bestimmung der Fließfähigkeit eines Knochenzementteigs (200), umfassend
ein hohlzylinderförmiges erstes Leitungselement (110) mit einem ersten Ende (111),
durch das ein Knochenzementteig (200) in das erste Leitungselement (110) einbringbar ist, und einem zweiten Ende (112),
wobei das zweite Ende (112) zumindest eine axial verlaufende Durchführung (120) aufweist, durch die der Knochenzementteig (200) aus dem ersten Leitungselement (110) förderbar ist,
**dadurch gekennzeichnet, dass**
die Durchführung (120) einen Innendurchmesser (125) im Bereich von 0,1 mm bis 3 mm und ein Verhältnis einer Länge (126) der Durchführung (120) zum Innendurchmesser (125) der Durchführung (120) von 10:1 bis 20:1 aufweist, damit bei einem Fördern des Knochenzementteigs (200) aus dem ersten Leitungselement (110) durch die Durchführung (120) mit einer Förderkraft von bis zu 2 kN bestimmbar ist, ob der Knochenzementteig (200) eine Fließfähigkeit aufweist, um in die Spongiosa eines Knochens applizierbar zu sein.

2. Vorrichtung (100) nach Anspruch 1, **dadurch gekennzeichnet, dass** die Durchführung (120) einen Innendurchmesser (125) im Bereich von 0,2 mm bis 2 mm, bevorzugt im Bereich von 0,3 mm bis 1 mm, aufweist.

3. Vorrichtung (100) nach einem der vorhergehenden Ansprüche, **dadurch gekennzeichnet, dass** die Vorrichtung (100) über das erste Ende (111) reversibel mit einem Vorratsbehälter (300) für Knochenzementteige (200) form- und/oder kraftschlüssig fluidleitend verbindbar ist.

4. Vorrichtung (100) nach Anspruch 3, **dadurch gekennzeichnet, dass** das erste Leitungselement (110) vom ersten Ende (111) in Richtung des zweiten Endes (112) konisch verjüngt ausgestaltet ist, so dass die Vorrichtung (100) über das erste Ende (111) reversibel durch Aufstecken mit einem Austragsschnorchel (310) eines Vorratsbehälters (300) für Knochenzementteige (200) form- und/oder kraftschlüssig fluidleitend verbindbar ist.

5. Vorrichtung (100) nach einem der vorhergehenden Ansprüche, **dadurch gekennzeichnet, dass** die Vorrichtung (100) einen separaten Kolben (130) umfasst, der ausgestaltet ist reversibel axial in dem ersten Leitungselement (110) verschiebbar zu sein, um einen Knochenzementteig (200) aus dem ersten Leitungselement (110) durch die zumindest eine Durchführung (120) zu fördern.

6. Vorrichtung (100) nach einem der vorhergehenden Ansprüche, **dadurch gekennzeichnet, dass** die Vorrichtung (100) ein zweites Leitungselement (140) aufweist, welches mit dem ersten Leitungselement (110) axial am zweiten Ende (112) fluidleitend verbunden ist, um einen aus dem ersten Leitungselement (110) durch die Durchführung (120) geförderten Knochenzementteig (200) aufzunehmen.

7. Verfahren (400) zur Bestimmung der Fließfähigkeit eines Knochenzementteigs (200) mit einer Vorrichtung (100), umfassend
ein hohlzylinderförmiges erstes Leitungselement (110) mit einem ersten Ende (111),
durch das ein Knochenzementteig (200) in das erste Leitungselement (110) einbringbar ist, und einem zweiten Ende (112), wobei das zweite Ende (112) zumindest eine axial verlaufende Durchführung (120) aufweist, durch die der Knochenzementteig (200) aus dem ersten Leitungselement (110) förderbar ist, wobei die Durchführung (120) einen Innendurchmesser (125) im Bereich von 0,1 bis 3 mm und ein Verhältnis einer Länge (125) der Durchführung (120) zum Innendurchmesser (125) der Durchführung (120) von 10:1 bis 20:1 aufweist,
**zumindest aufweisend die Schritte**
a) Bereitstellen eines Knochenzementteigs (200) in einem Vorratsbehälter (300),
b) Einbringen des Knochenzementteigs (200) aus dem Vorratsbehälter (300) durch das erste Ende (111) in das erste Leitungselement (110),
c) Ausüben einer Förderkraft von bis zu 2 kN, um den Knochenzementteig (200) aus Richtung des ersten Endes (111) durch die Durchführung (120) aus dem ersten Leitungselement (110) zu fördern, und
d) Überprüfen, ob der Knochenzementteig (200) durch die Durchführung (120) gefördert wird.

8. Verfahren (400) nach Anspruch 7, **dadurch gekennzeichnet, dass**
in Schritt b) das Einbringen des Knochenzementteigs (200) in das erste Leitungselement (110) durch ein reversibles fluidleitendes Verbinden der Vorrichtung (100) mit einem Austragsschnorchel (310) des Vorratsbehälters (300) und anschließendem Betätigen eines mit dem Vorratsbehälter verbundenen Austragselements erfolgt, um den Knochenzementteig (200) aus dem Vorratsbehälter (300) über den Austragsschnorchel (310) in das erste Leitungselement (110) zu fördern,
sowie
in Schritt c) die Förderkraft über eine fortgeführte Betätigung des Austragselements ausgeübt wird.

9. Verfahren (400) nach Anspruch 7, **dadurch gekennzeichnet, dass**
in Schritt b) das Einbringen des Knochenzementteigs (200) in das erste Leitungselement (110) durch eine Entnahme des Knochenzementteigs (200) aus dem Vorratsbehälter (300) und
ein Einfüllen des entnommenen Knochenzementteigs (200) durch das erste Ende (111) in das erste Leitungselement (110), sowie
in Schritt c) die Förderkraft mittels eines separaten Kolbens (130), der axial im ersten Leitungselement (110) verschoben wird, ausgeübt wird.
